# EUROPEAN PATENT APPLICATION

(11) **EP 2 433 569 A1**
(43) Date of publication of application: **28.03.2012**
(21) Application number: 11163080.2
(22) Date of filing: 19.04.2011
(51) Int. Cl.: A61B 8/08, G01S 15/89, G06T 7/00

(54) **3-dimensional (3D) ultrasound system using image filtering and method for operating 3D ultrasound system**

(30) Priority: 24.09.2010 KR 20100092822
(71) Applicant: Samsung Medison Co., Ltd., Nam-myun Hongchun-gun Kangwon do 250-875 (KR)
(72) Inventor: Lee, Kwang-Hee, 302-743, Daejeon (KR)
(74) Representative: Schmid, Wolfgang

(57) **Abstract**

Provided are a 3-dimensional (3D) ultrasound system using image filtering and a method for operating the 3D ultrasound system. The 3D ultrasound system using image filtering may include a scanning unit to generate a volume image of an object, a detecting unit to detect a contour line in each of 'n' first slice images extracted from the volume image, 'n' being a natural number, and a rendering control unit to correct the 'n' first slice images into 'n' second slice images by removing a non-feature region based on the contour line, and to combine and render the 'n' second slice images.

## Description

### CROSS-REFERENCE TO RELATED APPLICATION

This application claims the benefit of Korean Patent Application No. 10-2010-0092822, filed on September 24, 2010, in the Korean Intellectual Property Office, the disclosure of which is incorporated herein by reference.

### BACKGROUND

### 1. Field of the Invention

The present invention relates to a 3-dimensional (3D) ultrasound system and a method for operating the 3D ultrasound system, which may detect a contour line in a plurality of slice images extracted from a volume image of an object, and may correct the slice images based on the detected contour line, thereby providing a more accurate feature in the object.

### 2. Description of the Related Art

An ultrasound system is an apparatus for transmitting, from the surface of the body, an ultrasound wave signal toward a predetermined structure, that is, an object such as a fetus or an internal organ, inside the body, and for visualizing a cross section of soft tissues or a blood flow using information of the ultrasound wave signal reflected from the tissues of the body. The ultrasound system has advantages of a small size, a low cost, a real-time display, and a high stability without exposing patients or users to x-ray radiation, and thus, the ultrasound system is widely used along with other diagnostic imaging systems such as an x-ray diagnosis equipment, a computerized tomography (CT) scanner, a magnetic resonance imaging (MRI) equipment, a nuclear medicine diagnosis equipment, and the like.

When an anatomical structure other than the face of a fetus, for example, hands of the fetus or a placenta, is located over the face of the fetus, the ultrasound system has a difficulty in accurately checking the face of the fetus.

### SUMMARY

An aspect of the present invention provides a 3-dimensional (3D) ultrasound system and a method for operating the 3D ultrasound system, which may detect a contour line, for example, a facial line of a fetus, in a plurality of slice images extracted from a volume image of an object, may correct the slice images based on the detected contour line, and may combine and render the corrected slice images, thereby providing a more accurate feature in the object.

According to an aspect of the present invention, there is provided a 3D ultrasound system using image filtering including a scanning unit to generate a volume image of an object, a detecting unit to detect a contour line in each of 'n' first slice images extracted from the volume image, 'n' being a natural number, and a rendering control unit to correct the 'n' first slice images into 'n' second slice images by removing a non-feature region based on the contour line, and to combine and render the 'n' second slice images.

According to another aspect of the present invention, there is provided a method for operating a 3D ultrasound system using image filtering including generating a volume image of an object, detecting a contour line in each of 'n' first slice images extracted from the volume image, 'n' being a natural number, and correcting the 'n' first slice images into 'n' second slice images by removing a non-feature region based on the contour line, and combining and rendering the 'n' second slice images.

### EFFECT OF THE INVENTION

According to an aspect of the present invention, provided are a 3-dimensional (3D) ultrasound system and a method for operating the 3D ultrasound system, which may detect a contour line, for example, a facial line of a fetus, in a plurality of slice images extracted from a volume image of an object, may correct the slice images based on the detected contour line, and may combine and render the corrected slice images, thereby providing a more accurate feature in the object.

### BRIEF DESCRIPTION OF THE DRAWINGS

These and/or other aspects, features, and advantages of the invention will become apparent and more readily appreciated from the following description of exemplary embodiments, taken in conjunction with the accompanying drawings of which:
FIG. 1 is a block diagram illustrating a structure of a 3-dimensional (3D) ultrasound system using image filtering according to an embodiment of the present invention;
FIG. 2 is a view illustrating an example of providing of a more accurate feature in an object in the 3D ultrasound system using image filtering according to an embodiment of the present invention; and
FIG. 3 is a flowchart illustrating a method for operating the 3D ultrasound system using image filtering according to an embodiment of the present invention.

### DETAILED DESCRIPTION

Reference will now be made in detail to exemplary embodiments of the present invention, examples of which are illustrated in the accompanying drawings, wherein like reference numerals refer to the like elements throughout. Exemplary embodiments are described below to explain the present invention by referring to the figures.

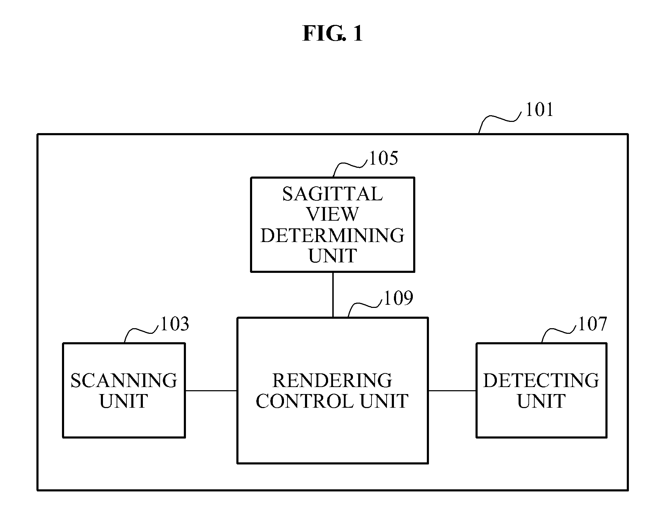

FIG. 1 is a block diagram illustrating a structure of a 3-dimensional (3D) ultrasound system 101 using image filtering according to an embodiment of the present invention.

Referring to FIG. 1, the 3D ultrasound system 101 using image filtering according to an embodiment of the present invention may include a scanning unit 103, a sagittal view determining unit 105, a detecting unit 107, and a rendering control unit 109.

The scanning unit 103 may generate a volume image of an object, for example, the face of a fetus, using an ultrasound wave signal.

The sagittal view determining unit 105 may determine a sagittal view by rotating the volume image. That is, the sagittal view determining unit 105 may adjust a front view of the volume image, for example, an image facing toward the eyes, nose, and mouth of a fetus, or may adjust a top view of the volume image, for example, an image facing down on the head of the fetus from the top, to be bilaterally symmetric, by rotating the volume image in a predetermined direction.

The detecting unit 107 may extract 'n' first slice images from the volume image, 'n' being a natural number. In this instance, the detecting unit 107 may extract first slice images by cutting the volume image determined for a sagittal view into 'n' slices in a predetermined direction. Here, the first slice image may be a top view of the volume image.

The detecting unit 107 may detect a contour line in each of the 'n' first slice images. In this instance, the detecting unit 107 may detect a contour line by extracting a boundary in the first slice image using at least one of a Haar-like feature, connection between points along the boundary, and a gradient. Here, the Haar-like feature is used to obtain information about a feature by summing pixel values in each region, adding weight values to the sums, and summing the results.

When the object is the face of a fetus, the detecting unit 107 may recognize, in the volume image, a face region, and a non-face region having a difference in brightness with the face region, using a Haar-like feature, and may detect a boundary between the face region and the non-face region as a contour line.

The rendering control unit 109 may correct the 'n' first slice images into 'n' second slice images by removing a non-feature region based on the contour line, may combine and render the 'n' second slice images, and may display the resulting combination on a display means, thereby providing a more accurate feature in the object.

In this instance, the rendering control unit 109 may correct the 'n' first slice images into 'n' second slice images by removing a non-feature region at an upper location based on the contour line.

When the object is the face of a fetus, the rendering control unit 109 corrects the 'n' first slice images into 'n' second slice images by removing a non-face region, that is, a region other than the face of the fetus, for example, hands of the fetus or a placenta, and may combine and render the 'n' second slice images, thereby providing a more accurate facial contour of the object.

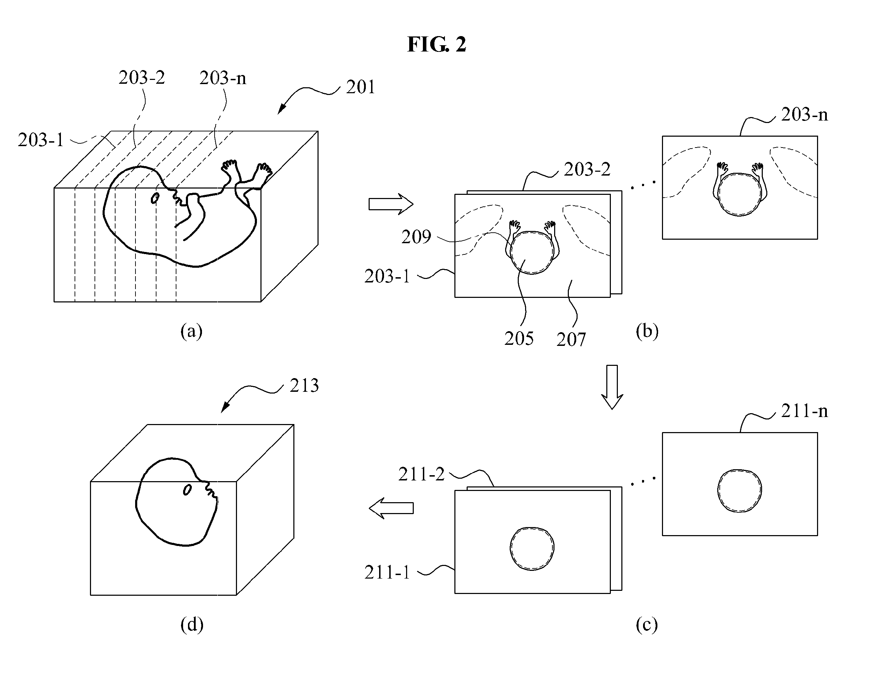

FIG. 2 is a view illustrating an example of providing of a more accurate feature in an object in the 3D ultrasound system using image filtering according to an embodiment of the present invention. Specifically, FIG. 2(a) is a view illustrating a volume image of an object, FIG. 2(b) is a view illustrating first slice images extracted from the volume image, FIG. 2(c) is a view illustrating second slice images produced by correcting the first slice images, and FIG. 2(d) is a view illustrating a volume image obtained by combining the second slice images.

Referring to FIG. 2, the 3D ultrasound system may generate a volume image of an object, may detect a contour line in each of 'n' first slice images extracted from the volume image, and may correct the 'n' first slice images into 'n' second slice images by removing a non-feature region based on the contour line, 'n' being a natural number. The 3D ultrasound system may combine and render the 'n' second slice images and may display the resulting combination on a display means, thereby providing a more accurate feature in the object.

For example, when the object is the face of a fetus, the 3D ultrasound system may generate a volume image 201 of the object, may recognize a face region 205 and a non-face region 207 having a difference in brightness with the face region 205 in each of 'n' first slice images #1 203-1 to #n 203-n extracted from the volume image 201, and may detect a boundary 209 between the face region 205 and the non-face region 207 as a contour line.

The 3D ultrasound system may correct the 'n' first slice images #1 203-1 to #n 203-n into 'n' second slice images #1 211-1 to #n 211-n by removing the non-face region 207 based on the boundary 209 corresponding to a contour line.

The 3D ultrasound system may generate a volume image 213 by combining and rendering the 'n' second slice images #1 211-1 to #n 211-n, and may display the volume image 213 on a display means, thereby providing a more accurate feature in the object, that is, a more accurate face of the fetus.

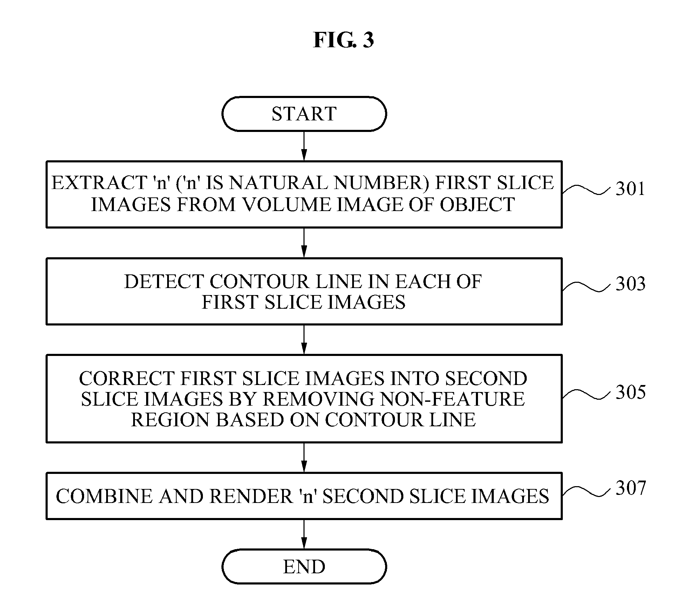

FIG. 3 is a flowchart illustrating a method for operating the 3D ultrasound system using image filtering according to an embodiment of the present invention.

Referring to FIG. 3, the 3D ultrasound system may extract 'n' first slice images from a volume image of an object, in operation 301, 'n' being a natural number.

That is, the 3D ultrasound system may generate a volume image of an object, for example, the face of a fetus, using an ultrasound wave signal, may determine a sagittal view by rotating the volume image, and may extract 'n' first slice images by cutting the volume image determined for a sagittal view into 'n' slices in a predetermined direction. In this instance, the first slice image may be a top view of the volume image, for example, an image facing down on the fetal head from the top.

In operation 303, the 3D ultrasound system may detect a contour line in each of the 'n' first slice images. In this instance, the 3D ultrasound system may detect a contour line by extracting a boundary in the first slice image using at least one of a Haar-like feature, connection between points along the boundary, and a gradient.

When the object is the face of a fetus, the 3D ultrasound system may recognize, in the volume image, a face region and a non-face region having a difference in brightness with the face region, using a Haar-like feature, and may detect a boundary between the face region and the non-face region as a contour line.

In operation 305, the 3D ultrasound system may correct the 'n' first slice images into 'n' second slice images by removing the non-feature region based on the contour line.

In this instance, the 3D ultrasound system may correct the 'n' first slice images into 'n' second slice images by removing a non-feature region at an upper location based on the contour line.

When the object is the face of a fetus, the 3D ultrasound system may correct the 'n' first slice images into 'n' second slice images by removing a non-face region, that is, a region other than the face of the fetus, for example, hands of the fetus or a placenta, so that a more accurate facial contour of the fetus may be provided when the 'n' second slice images are combined and rendered.

In operation 307, the 3D ultrasound system may combine and render the 'n' second slice images.

That is, the 3D ultrasound system may combine and render the 'n' second slice images, and may display the resulting combination on a display means, thereby providing a more accurate feature in the object.

According to an embodiment of the present invention, the 3D ultrasound system may detect a contour line in a plurality of slice images extracted from a volume image of an object, may correct the slice images based on the detected contour line, for example, a facial line of a fetus, and may combine and render the corrected slice images, thereby providing a more accurate feature in an object.

The above-described exemplary embodiments of the present invention may be recorded in non-transitory computer-readable media including program instructions to implement various operations embodied by a computer. The media may also include, alone or in combination with the program instructions, data files, data structures, and the like. Examples of non-transitory computer-readable media include magnetic media such as hard disks, floppy disks, and magnetic tape; optical media such as CD ROM disks and DVDs; magneto-optical media such as optical disks; and hardware devices that are specially configured to store and perform program instructions, such as read-only memory (ROM), random access memory (RAM), flash memory, and the like. Examples of program instructions include both machine code, such as produced by a compiler, and files containing higher level code that may be executed by the computer using an interpreter. The described hardware devices may be configured to act as one or more software modules in order to perform the operations of the above-described exemplary embodiments of the present invention, or vice versa.

Although a few exemplary embodiments of the present invention have been shown and described, the present invention is not limited to the described exemplary embodiments. Instead, it would be appreciated by those skilled in the art that changes may be made to these exemplary embodiments without departing from the principles and spirit of the invention, the scope of which is defined by the claims and their equivalents.

## Claims

1. A 3-dimensional (3D) ultrasound system using image filtering, the system comprising:
a scanning unit to generate a volume image of an object;
a detecting unit to detect a contour line in each of 'n' first slice images extracted from the volume image, 'n' being a natural number; and
a rendering control unit to correct the 'n' first slice images into 'n' second slice images by removing a non-feature region based on the contour line, and to combine and render the 'n' second slice images.

2. The system of claim 1, wherein the detecting unit detects the contour line in the first slice image using at least one of a Haar-like feature, a connection between points along the boundary, and a gradient.

3. The system of claim 2, wherein when the object is the face of a fetus, the detecting unit recognizes, in the volume image, a face region and a non-face region having a difference in brightness with the face region using the Haar-like feature, and detects a boundary between the face region and the non-face region as the contour line.

4. The system of claim 3, wherein the rendering control unit removes, as the non-feature region, the non-face region related to hands of the fetus or a placenta other than the face of the fetus.

5. The system of claim 1, further comprising:
a sagittal view determining unit to determine a sagittal view by rotating the volume image,
wherein the detecting unit extracts the first slice images by cutting the volume image determined for the sagittal view into 'n' slices in a predetermined direction.

6. The system of claim 1, wherein the rendering control unit corrects the first slice images into the second slice images by removing the non-feature region at an upper location based on the contour line.

7. A method for operating a 3-dimension (3D) ultrasound system using image filtering, the method comprising:
generating a volume image of an object;
detecting a contour line in each of 'n' first slice images extracted from the volume image, 'n' being a natural number; and
correcting the 'n' first slice images into 'n' second slice images by removing a non-feature region based on the contour line, and combining and rendering the 'n' second slice images.

8. The method of claim 7, wherein the detecting of the contour line comprises detecting the contour line in the first slice image using at least one of a Haar-like feature, connection between points along the boundary, and a gradient.

9. The method of claim 8, wherein when the object is the face of a fetus, the detecting of the contour line in the first slice image using the Haar-like feature comprises recognizing, in the volume image, a face region and a non-face region having a difference in brightness with the face region using the Haar-like feature, and detecting a boundary between the face region and the non-face region as the contour line.

10. The method of claim 9, wherein the combining and rendering of the second slice images comprises removing, as the non-feature region, the non-face region related to hands of the fetus or a placenta other than the face of the fetus.

11. The method of claim 7, further comprising:
determining a sagittal view by rotating the volume image; and
extracting the first slice images by cutting the volume image determined for the sagittal view into 'n' slices in a predetermined direction.

12. The method of claim 7, wherein the combining and rendering of the second slice images comprises correcting the first slice images into the second slice images by removing the non-feature region at an upper location based on the contour line.
